# EUROPÄISCHE PATENTSCHRIFT

(11) **EP 1 753 482 B1**
(45) Veröffentlichungstag und Bekanntmachung des Hinweises auf die Patenterteilung: **11.01.2012**
(21) Anmeldenummer: 05742280.0
(22) Anmeldetag: 26.05.2005
(51) Int. Cl.: A61M 1/06, A61J 9/00

(54) **EINWEG-BRUSTHAUBENSET**
DISPOSABLE BREAST CUP SET
ENSEMBLE EMBOUT DE TIRE-LAIT JETABLE

(30) Priorität: 03.06.2004 WO PCT/CH2004/000334
(43) Veröffentlichungstag der Anmeldung: 21.02.2007
(73) Patentinhaber: Medela Holding AG, 6340 Baar (CH)
(72) Erfinder: KREBS, Yvonne, CH-8136 Gattikon (CH); VÖGELIN, Stefan, CH-5644 Auw (CH); STADELMANN, Urs, CH-5735 Pfeffikon (CH)
(74) Vertreter: Clerc, Natalia
(86) Internationale Anmeldenummer: PCT/CH2005/000298
(87) Internationale Veröffentlichungsnummer: WO 2005/118023

(56) Entgegenhaltungen:
- GB-A- 550 187
- US-A- 6 110 140
- US-A1- 2003 230 351
- US-A1- 2004 087 898
- US-B1- 6 461 324

## Beschreibung

### Technisches Gebiet

Die Erfindung betrifft ein Einweg-Brusthaubenset gemäss Oberbegriff des Patentanspruchs 1.

### Stand der Technik

Derartige Brusthaubensets werden zusammen mit Brustpumpen zum Abpumpen von menschlicher Muttermilch eingesetzt. Sie bestehen üblicherweise aus einem Brusthaubentrichter, welcher über ein Brusthaubenanschlussteil mit einem Kopplungsteil verbunden ist. Das Kopplungsteil lässt sich auf einen Milchsammelbehälter, vorzugsweise eine Milchbabyflasche aus Glas oder Kunststoff aufschrauben. Das Brusthaubenanschlussteil ist entweder mit einem mechanischen Pumpmechanismus verbindbar oder es weist einen Anschlussstutzen für einen Verbindungsschlauch auf, welcher an eine elektrische Brustpumpe angeschlossen werden kann. Ein derartiges Brusthaubenset ist beispielsweise in US-A-6'461'324 beschrieben.

Diese Brusthaubensets sind üblicherweise aus Kunststoff gefertigt. Oft sind sie sterilisierbar und autoklavierbar und lassen sich somit mehrfach verwenden. Erhöhte Anforderungen an die Hygiene sowohl im Spital- wie auch im Privatgebrauch haben jedoch in letzter Zeit das Bedürfnis nach einem wegwerfbaren und deshalb kostengünstigen Brusthaubenset verstärkt.

Es sind zwar wegwerfbare Sets bekannt. Diese lassen sich jedoch trotzdem mehrfach verwenden, was zum Teil aus Kostengründen auch getan wird. Ferner offenbart US-A-6'575'202 ein Brusthaubenset mit einem Sammelbehälter, bei welchem zwar der Sammelbehälter weggeworfen wird, jedoch nicht die restlichen Elemente.

### Darstellung der Erfindung

Es ist deshalb eine Aufgabe der Erfindung, ein Brusthaubenset zu schaffen, welches den erhöhten Hygieneanforderungen genügt und trotzdem kostengünstig herstellbar ist.

Diese Aufgabe löst ein Einweg-Brusthaubenset mit den Merkmalen des Patentanspruchs 1.

Das erfindungsgemässe Brusthaubenset weist ein Kopplungsteil zur Verbindung mit einem Milchsammelbehälter und ein Brusthaubenanschlussteil zur Verbindung mit einem Brusthaubentrichter auf, wobei das Kopplungsteil und das Brusthaubenanschlussteil miteinander über eine irreversibel zerstörbare Verbindung in Form eines Garantieelements miteinander verbunden sind. Das Garantieelement ist dabei derart ausgestaltet, dass bei einem erstmaligen Trennen von Kopplungsteil und Brusthaubenanschlussteil oder bei erstmaligem Entfernen die Verbindung des Brusthaubenanschlussteils und des Kopplungsteils irreversibel zerstört wird. Dies kann z.B. durch Entnahme, Entfernen, Aufreissen, Ablösen o.ä. des Garantieelementes geschehen.

Das Kopplungsteil und das Brusthaubenanschlussteil lassen sich nach erstrnaliger Trennung nicht oder zumindest nicht genügend gut wieder miteinander verbinden. Da eine erstrnalige Trennung notwendig ist, um die Milch zu lagern bzw, die Milch zu verabreichen, lassen sich beide Teile nur ein einziges Mal zum Abpumpen der Milch verwenden.

Unter dem Begriff Garantieelement ist somit im vorliegenden Fall ein Element zu verstehen, welches bei der unbenutzten Vorrichtung, das heisst beim unbenutzten Brusthaubenset, in intakter Form vorliegt und normalerweise beim Abpumpen der Muttermilch auch in diesem Zustand belassen wird.

In dem Moment aber, wo die Benutzerin mit dem Abpumpen abgeschlossen hat oder der Sammelbehälter voll ist, und die im Sammelbehälter aufgefangene Muttermilch verwenden oder aufbewahren will, ist es, zum Entfernen des oberen Teiles des Brusthaubensets erforderlich, dieses Garantieelement zu manipulieren. Das Garantieelement muss, um überhaupt die Möglichkeit zu erhalten, den oberen Teil, das heisst dass Brusthaubenanschlussteil, zu entfernen, in einer die Verbindung irreversibel aufbrechenden oder aufreissenden Weise manipuliert werden, so dass es dabei gewissermassen zerstört wird.

Zu diesem Zweck verfügt das Garantieelement z.B. über eine Sollbruchstelle, welche durchbrochen oder aufgerissen wird, um die Teile voneinander zu trennen. Vorzugsweise ist zusätzlich die Verbindung zwischen dem Brusthaubenanschlussteil und dem Kopplungsteil derart ausgestaltet, dass nach Aufbrechen des Garantieelementes diese beiden Teile nicht mehr ohne weiteres miteinander verbunden werden können.

Es muss hervorgehoben werden, dass unter dem Garantieelement nicht irgendein beliebiges Element, welches unter Anwendung von Gewalt zerstört werden kann, verstanden werden soll. Es handelt sich beim hier gemeinten Garantieelement um ein Element, welches bei der vorgesehenen Handhabung des Brusthaubensets irreversibel zerstört wird, d.h. bei welchem die irreversible Zerstörung des Elementes neben einer ggf, vorhandenen Verbindungsfunktion zwischen Brusthaubenanschlussteil und dem Kopplungsteil eigentlicher Sinn und Zweck ist und bei bestimmungsgemässer Handhabung auftritt.

Dies äussert sich in struktureller Hinsicht darin, dass es ohne irreversible Zerstörung des Garantieelementes eben gar nicht möglich ist, Brusthaubenanschlussteil und Kopplungsteil voneinander zu trennen und den Inhalt des Sammelbehälters z.B. zu entnehmen oder diesen ohne Aufsatz aufzubewahren. Kopplungsteil und Brusthaubenanschlussteil können somit bestimmungsgemäss nur voneinander getrennt werden, wenn das Garantieelement vorgängig irreversibel zerstört wird. Es können auch mehrere Garantieelemente vorgesehen werden.

Es handelt sich also beim Garantieelement im Sinne der Erfindung nicht um ein Element, welches an sich für eine solche Garantiefunktion nicht vorgesehen ist und welches höchstens bei bestimmungsfremder gewaltsamer Manipulation irreversibel zerstört wird.

Vergleichbar ist dieses Garantieelement beispielsweise mit einem Siegel, und entsprechend kann es sich zum Beispiel um Papierstreifen, Kunststoffstreifen, oder entsprechende -bänder, welche an einer Schnittlinie zwischen dem Brusthaubenanschlussteil und dem Kopplungsteil vorgesehen sind, handeln. Das Garantieelement kann so zum Beispiel als umlaufender Papierstreifen am Brusthaubenanschlussteil und dem Kopplungsteil angeklebt sein.

In einer einfachsten Ausführungsform umfasst das erfindungsgemässe Brusthaubenset im Sinne dieser Erfindung das Kopplungsteil, das Brusthaubenanschlussteil und das Garantieelement, wobei Kopplungsteil und Brusthaubenanschlussteil mit dem Brusthaubentrichter bzw. dem Milchsammelbehälter lös- oder unlösbar verbindbar sind bzw. bereits verbunden sind. Je nach Ausführungsform kann das Brusthaubenset noch weitere Elemente aufweisen. Beispielsweise lassen sich Kopplungsteil und Brusthaubenanschlussteil einstückig mit weiteren Teilen, insbesondere dem Sammelbehälter bzw. der Brusthaube, ausbilden.

Ist das Kopplungsteil nicht zerstörungsfrei lösbar mit einem Milchsammelbehälter verbunden bzw. einstückig an diesem angeformt, so ist auch gewährleistet, dass der Milchsammelbehälter nur noch zur Lagerung oder Verabreichung der Milch eingesetzt wird und ebenfalls nicht nochmals zum Sammeln neuer Milch eingesetzt werden kann.

Vorzugsweise ist das Kopplungsteil als separates Teil ausgebildet und wird erst kurz vor Gebrauch mit dem Sammelbehälter verbunden. Dadurch lässt sich der Sammelbehälter becherförmig gestalten und somit stapeln. Diese Ausführungsform weist den Vorteil auf, dass der Platzbedarf beim Transport und Lagern des Brusthaubensets geringer ist als bei einem einstückigen oder bereits zusammengesetzten Set.

Vorzugsweise ist der Brusthaubentrichter mit dem auf die Brust aufgelegten Trichterelement lösbar mit dem Brusthaubenanschlussteil verbindbar. Vorzugsweise lässt sich der Brusthaubentrichter in das Brusthaubenanschlussteil einstecken. Dies weist ebenfalls den Vorteil auf, dass der Platzbedarf beim Transport und Lagern verringert ist. Des weiteren lassen sich je nach Form und Grösse der Brust verschiedene Brusthaubentrichterformen und -grössen verwenden, ohne dass das restliche Set angepasst sein muss. Dies verringert die Herstellungskosten und spart wiederum Lagerplatz.

In bevorzugten Ausführungsformen ist das Brusthaubenset mit einem Einwegventil versehen, welches das Totvolumen im Set verringert und somit die Effizienz des Saugvorgangs erhöht.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1a: eine Seitenansicht eines erfindungsgemässen Brusthaubensets in einer ersten Ausführungsform;
- Figur 1b: das Brusthaubenset gemäss Figur 1a in einer Explosionsdarstellung;
- Figur 1c: eine vergrösserte Darstellung eines Teils der Figur 1b;
- Figur 1d: eine Längsschnitt durch einen oberen Teil des Brusthaubensets gemäss Figur 1 a;
- Figur 1e: eine perspektivische Darstellung des oberen Teils gemäss Figur 1d;
- Figur 1f: eine Ansicht des oberen Teils gemäss Figur 1e von einer ersten Seite;
- Figur 1g: eine Ansicht des oberen Teils gemäss Figur 1e von einer zweiten Sei-
- Figur 1h: te; eine perspektivische Darstellung des Brusthaubensets gemäss Figur 1 a nach Entfernen des Garantieelements;
- Figur 1i: den Sammelbehälter gemäss Figur 1a mit einem Verschlussdeckel in einer Explosionsdarstellung;
- Figur 1k: eine vergrösserte Darstellung des Einwegventils gemäss Figur 1a;
- Figur 2a: eine perspektivische Darstellung eines erfindungsgemässen Brusthaubensets in einer zweiten Ausführungsform in einer Explosionsdarstellung;
- Figur 2b: das Brusthaubenset gemäss Figur 2a im zusammengesetzten Zustand;
- Figur 2c: ein vergrösserter Ausschnitt gemäss Figur 2b;
- Figur 3a: eine Seitenansicht eines erfindungsgemässen Brusthaubensets in einer dritten Ausführungsform;
- Figur 3b: das Brusthaubenset gemäss Figur 3a in einer Explosionsdarstellung;
- Figur 3c: den Milchsammelbehälter gemäss Figur 3a mit einem Verschlussdeckel;
- Figur 4: einen Längsschnitt durch einen oberen Teil eines erfindungsgemässen Brusthaubensets in einer vierten Ausführungsform;
- Figur 5a: eine perspektivische Darstellung eines oberen Teils eines erfindungsgemässen Brusthaubensets in einer fünften Ausführungsform in einer Explosionsdarstellung;
- Figur 5b: einen Längsschnitt durch den Teil gemäss Figur 5a;
- Figur 6: eine perspektivische Darstellung eines Ventils gemäss einer sechsten Ausführungsform;
- Figur 7a: eine perspektivische Darstellung eines Ventils gemäss einer siebten Ausführungsform von einer ersten Seite;
- Figur 7b: eine perspektivische Darstellung des Ventils gemäss Figur 7a von einer zweiten Seite;
- Figur 7c: einen Längsschnitt durch das Ventil gemäss Figur 7a;
- Figur 8a: eine perspektivische Darstellung eines Ventils gemäss einer achten Ausführungsform;
- Figur 8b: eine Ansicht des Ventils gemäss Figur 8a;
- Figur 9a: eine perspektivische Darstellung eines Ventils gemäss einer neunten Ausführungsform von einer ersten Seite;
- Figur 9b: eine perspektivische Darstellung des Ventils gemäss Figur 9a von einer zweiten Seite und
- Figur 9c: einen Längsschnitt durch das Ventil gemäss Figur 9a.

### Wege zur Ausführung der Erfindung

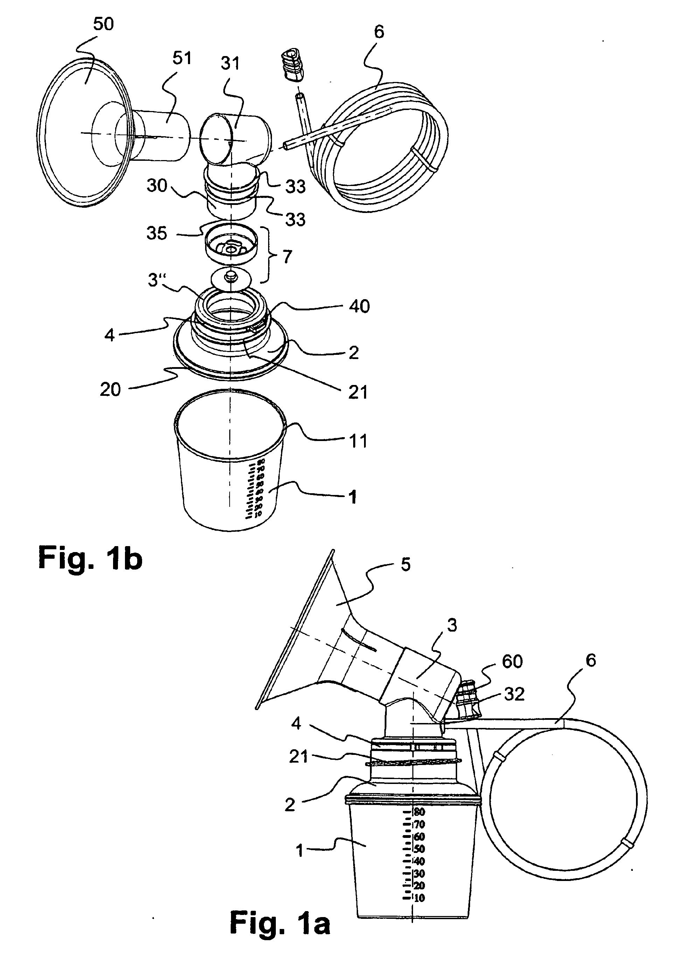

In den Figuren 1a bis 1k ist ein erstes Ausführungsbeispiel des erfindungsgemässen wegwerfbares Brusthaubensets dargestellt. Die einzelnen Teile sind vorzugsweise aus Kunststoff gefertigt.

Wie in Figur 1a dargestellt, ist ein Milchsammelbehälter 1 über ein Kopplungsteil 2 mit einem Brusthaubenanschlussteil 3 verbunden. Im Brusthaubenanschlussteil 3 ist ein Brusthaubentrichter 5 gehalten, welcher beim Abpumpen der Muttermilch an eine Mutterbrust angelegt wird. Das Brusthaubenanschlussteil 3 weist einen pumpenseitigen Anschluss 32 auf, an welchem ein erstes Ende eines Verbindungsschlauchs 6 angeschlossen werden kann. Ein zweites Ende 60 des Verbindungsschlauchs 6 lässt sich mit einer hier nicht dargestellten elektrischen oder mechanischen Brustpumpe oder Zentralvakuum (das heisst eine fertig installierte Absauganlage) verbinden. Die Brustpumpe erzeugt in der Brusthaube in einem zyklischen Rhythmus einen Unterdruck, so dass die Brust stimuliert wird und Milch durch das Brusthaubenanschlussteil 3 in den Sammelbehälter 1 gelangen kann.

Vorzugsweise ist im Bereich zwischen Brusthaubenanschlussteil 3 und Sammelbehälter 1 ein Rückschlagventil 7 vorhanden, wie dies in Figur 1b erkennbar ist. Dieses Ventil 7 verschliesst die behälterseitige Einlassöffnung 35 des Brusthaubenanschlussteils 3 und verringert so das Totvolumen, welches mittels der Pumpe evakuiert werden muss, auf ein Minimum.

Erfindungsgemäss ist ein Garantieelement 4 vorhanden, welches das Brusthaubenanschlussteil 3 und das Kopplungsteil 2 miteinander verbindet. Als Garantieelement eignet sich jegliche Verbindung, welche bei einem erstmaligen Trennen dieser zwei Teile bzw. bei einem erstmaligen Entfernen die Verbindung des Brusthaubenanschlussteil 3 und des Kopplungsteils 2 irreversibel zerstört.

Vorzugsweise ist dieses Garantieelement 4 eine Reissicherung, beispielsweise ein umlaufendes Band, welches über dünne, bei der Trennung zerreissende Stege 41 mit dem Kopplungsteil 2 und dem Brusthaubenanschlussteil 3 verbunden ist. Üblicherweise ist das Garantieelement 4 ebenfalls aus Kunststoff gefertigt und an einem der zwei Teile bzw. an beiden Teile 2, 3 angespritzt.

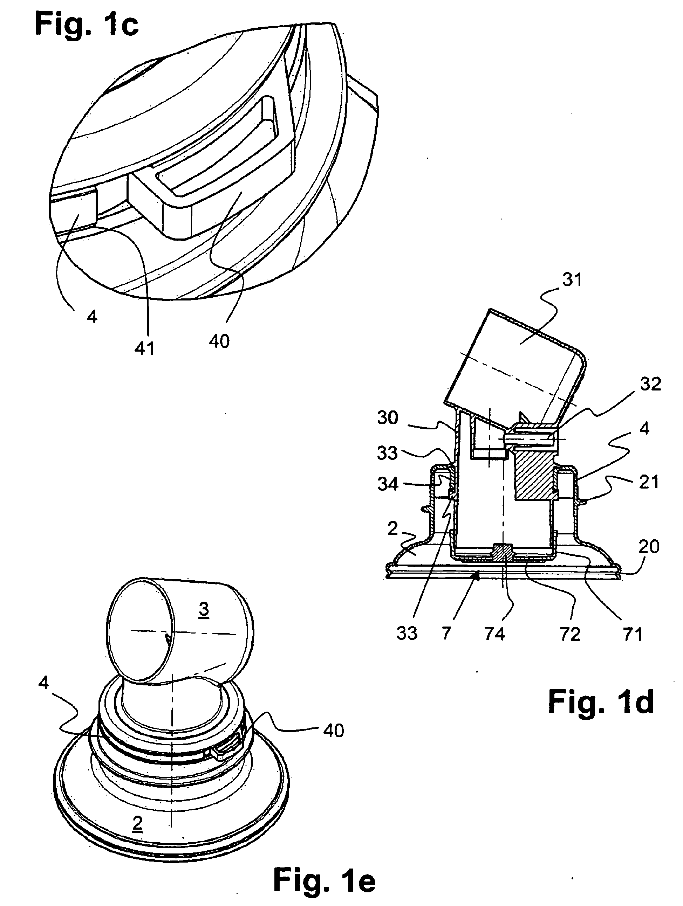

Das Garantieelement 4 weist vorzugsweise eine vorstehende Lasche 40 auf, damit es von Hand gut ergriffen werden kann und sich einfach aufreissen lässt. Dies ist in Figur 1c dargestellt.

Die einzelnen oben erwähnten Teile des Brusthaubensets können ein- oder mehrstückig ausgebildet sein. Es lassen sich auch zwei oder mehrere dieser Teile gemeinsam einstückig ausgebilden.

Wie in Figur 1b erkennbar ist, besteht das erste Ausführungsbeispiel aus mehreren Einzelteilen. Der Sammelbehälter 1 ist ein zylinderförmiger Becher, welcher an seinem oberen Rand ein erstes unteres Schnappverschlusselement 11 in Form einer umlaufenden Wulst aufweist. Das Kopplungsteil 2 weist an seinem unteren Rand ein erstes oberes Schnappverschlusselement 20 in Form einer umlaufenden Rastklinke auf. Werden diese zwei Elemente zusammengesteckt, umfasst die Rastklinke die Wulst und bildet somit eine feste Verbindung zwischen Kopplungsteil 2 und Sammelbehälter 1.

Das Brusthaubenanschlussteil 3 ist in dieser Ausführungsform zweistückig ausgebildet. Er weist ein oberes Element 3' mit einem behälterseitigen Anschlusstutzen 30 und einem trichterseitigen Anschlussstutzen 31 auf. Der Brusthaubentrichter 5 weist ein Trichterelement 50 zur Auflage an die Brust und einen Trichterstutzen 51 auf, welcher fest verbunden oder in den trichterseitigen Anschlussstutzen 31 einsteckbar und auch wieder einfach aus diesem entfernbar ist.

Ein unteres Element 3" des Brusthaubenanschlussteils 3 ist über das Garantieelement 4 mit dem Kopplungsteil 2 verbunden.

Das Ventil 7, hier bestehend aus einer Ventilmembran 70 und einem Ventilkörper 71, ist vorzugsweise auf dem unteren Rand des behälterseitigen Anschlussstutzens 30 aufgesteckt, wie dies in Figur 1d gut erkennbar ist.

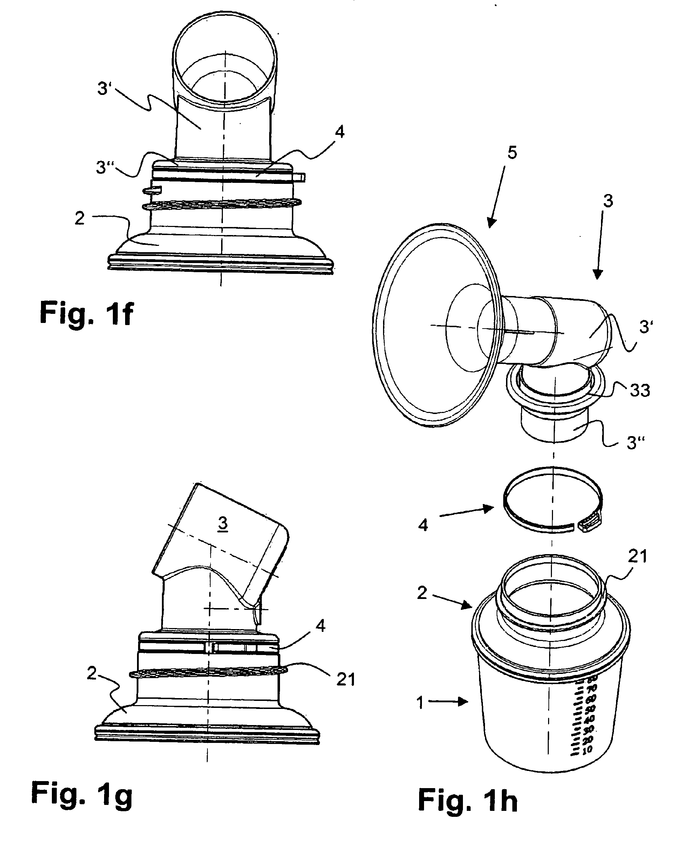

Am behälterseitigen Anschlusstutzen 30 ist ein zweites oberes Schnappverschlusselement 33 angeordnet, dessen Gegenstück 34 am unteren Element 3" angeformt ist. Dies ist in Figur 1d gut sichtbar. Das zweite obere Schnappverschlusselement 33 wird vorzugsweise durch eine obere und untere umlaufende Rastklinke gebildet, welche sich an einen in die Öffnung des Kopplungsteils 2 hineinragenden Stutzen festhaken. Diese Verbindung zwischen dem oberen und dem unteren Element 3', 3" ist vorzugsweise nicht zerstörungsfrei lösbar, so dass, wie in den Figuren 1e, 1f und 1g erkennbar ist, Kopplungsteil 2 und Brusthaubenanschlussteil 3 eine über das Garantieelement 4 verbundene Einheit bilden.

Wird nun das Garantieband 4 entfernt, so löst sich das gesamte Brusthaubenanschlussteil 3, d.h. das obere und das untere Element 3', 3", vom Kopplungsteil 2, wie dies in Figur 1h erkennbar ist. Durch diese Trennung lässt sich beim erneuten Verbinden der Teile keine genügend gute Verbindung mehr herstellen, so dass diese Kombination nicht nochmals zum Abpumpen verwendet werden kann.

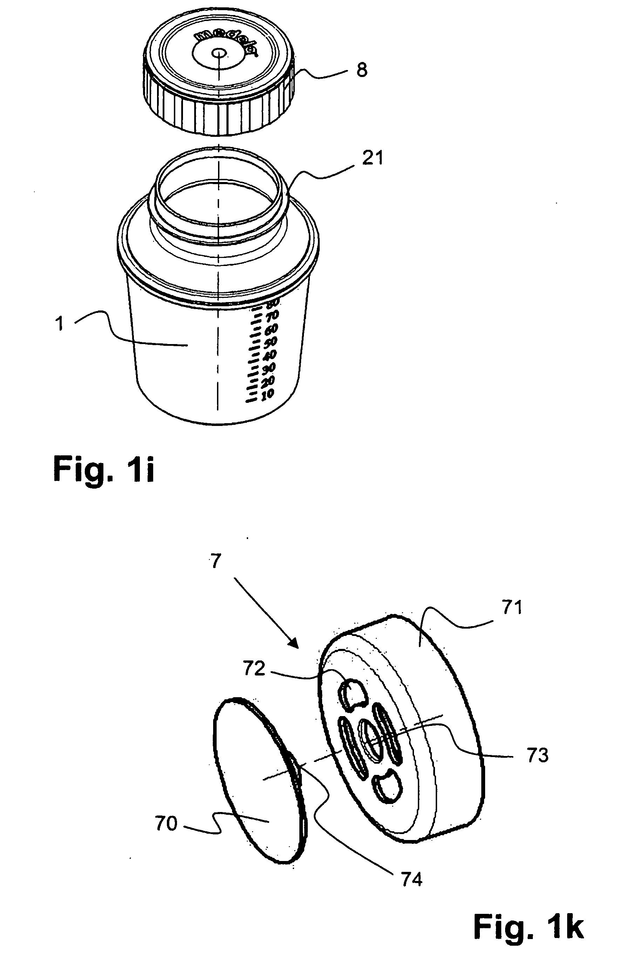

Zurück bleibt jedoch ein becherförmige Sammelbehälter 1 mit einem aufgesetzten Kopplungsteil 2, welche zusammen eine Babyflasche bilden. Das Kopplungsteil 2 bildet einen Flaschenhalt. Da es unterhalb der Befestigungsstelle des Garantieelements 4 mit einem Aussengewinde 21 versehen ist, lässt sich die Flasche bis zu ihrem Gebrauch mittels eines Deckels 8 verschliessen. Dies ist in Figur 1 i dargestellt. Bei Gebrauch lässt sich der Deckel 8 entfernen und es kann in bekannter Weise ein Aufsatz mit einem Sauger aufgesetzt werden.

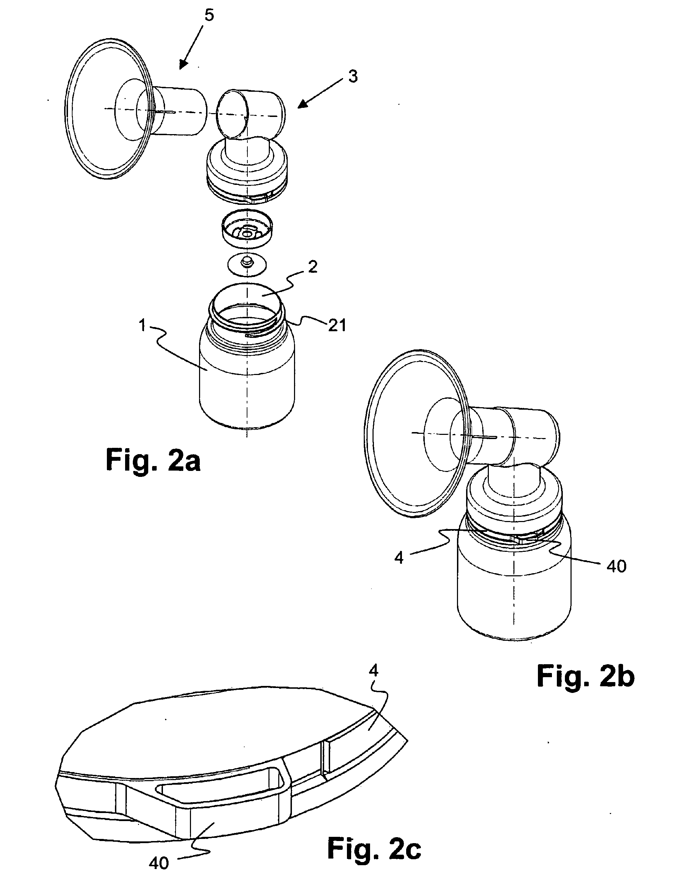

In einer zweiten Ausführungsform des erfindungsgemässen Brusthaubensets, dargestellt in den Figuren 2a bis 2c, ist das Brusthaubenanschlussteil 3 einstückig ausgebildet und das Garantieelement 4 an ihm angeformt. Auch hier ist wiederum mittels eines Schnappverschlusses und des Garantiebandes eine irreversible lösbare Verbindung mit dem Kopplungsteil 2 vorhanden. In dieser Ausführungsform sind der Sammelbehälter 1 und das Kopplungsteil 2 durch eine einstückige Flasche gebildet. In einer anderen Variante sind sie jedoch gemäss Figur 1b zweistückig ausgebildet. Der Brusthaubentrichter 5 lässt sich auch bei diesem Beispiel bei Gebrauch in das Brusthaubenanschlussteil 3 einschieben.

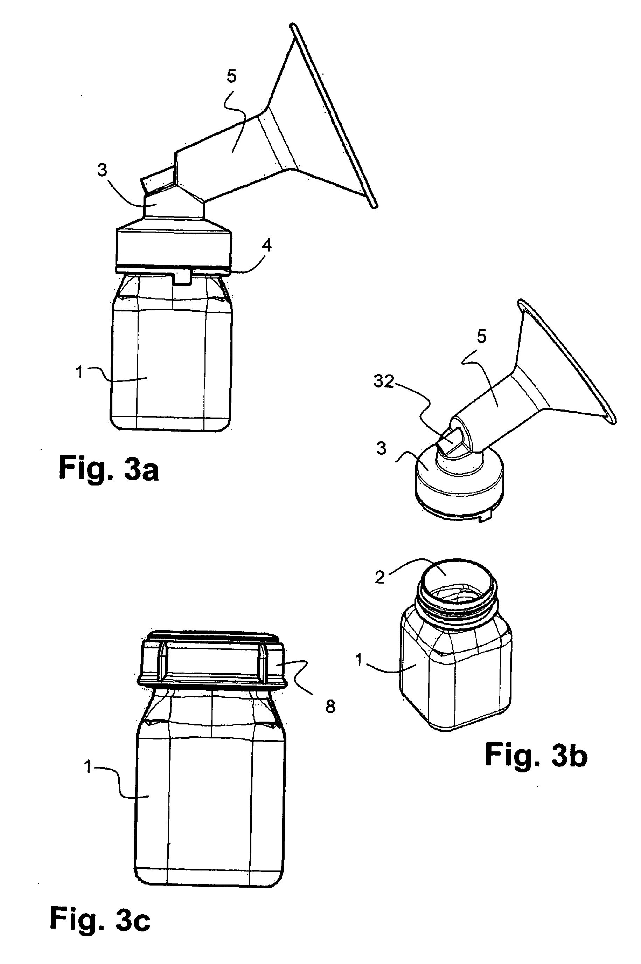

Im Ausführungsbeispiel gemäss den Figuren 3a bis 3c sind der Brusthaubentrichter 5 und das Brusthaubenanschlussteil 3 gemeinsam einstückig ausgebildet. Das Garantieelement 4 ist an der unteren Kante des Brusthaubenanschlussteils 3 angeformt. Auch in diesem Beispiel kann der Sammelbehälter 1 und das Kopplungsteil 2 durch eine einstückige Flasche gebildet sein oder sie können getrennte Teile bilden. In diesem Beispiel weist der Sammelbehälter 1 nicht einen runden sondern einen rechteckigen, insbesondere einen quadratischen Querschnitt auf. Dadurch lässt er sich platzsparender lagern als runde Flaschen. Selbstverständlich können auch die oben beschriebenen zweiteiligen Flaschen eine derartige rechteckige oder quadratische Fonn aufweisen.

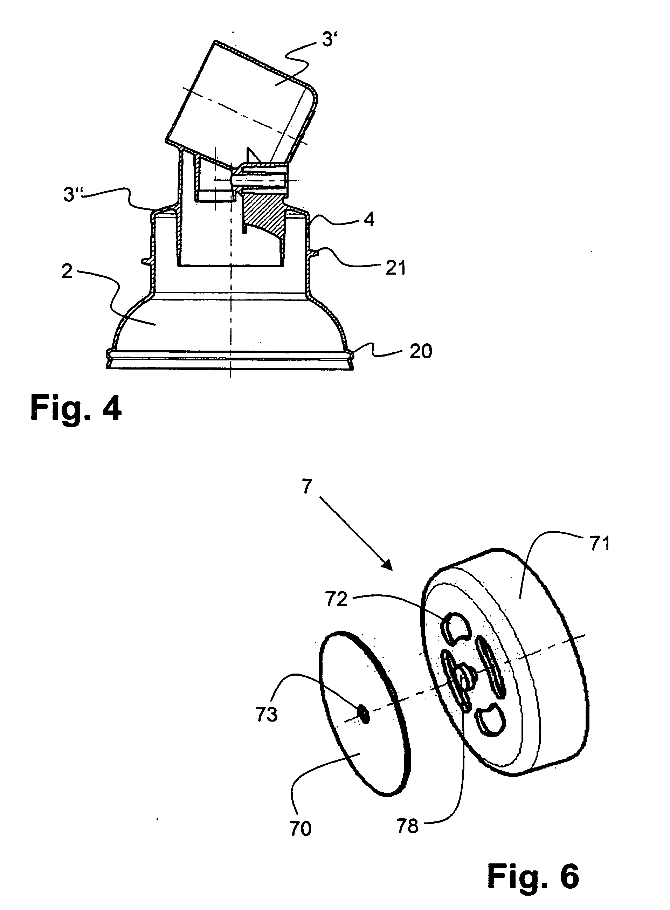

Die Ausführungsform gemäss Figur 4 zeigt ein Brusthaubenanschlussteil und ein Kopplungsteil, welche einstückig miteinander verbunden sind. Auch in diesem Fall ist das Garantieelement angespritzt.

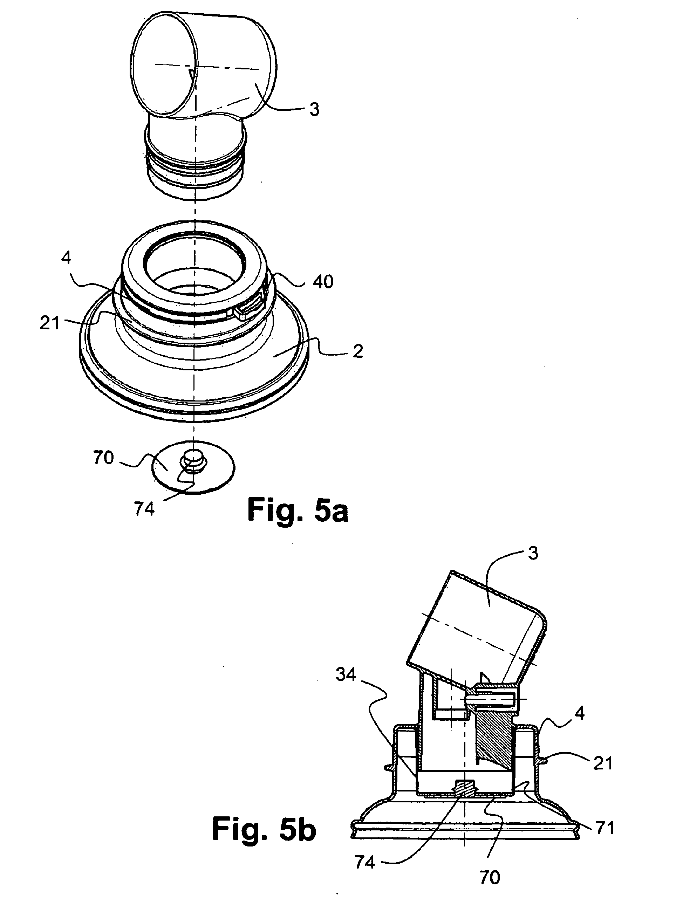

In der Ausführungsform gemäss den Figuren 5a und 5b ist das Ventil 7 nicht am behälterseitigen Anschlussstutzen 30 sondern an einem in das Kopplungsteil 2 hineinragenden, stutzenförmigen Gegenstück 34 angeordnet. Der Ventilkörper 71 kann aufgesteckt oder einstückig an diesem Gegenstück 34 angeformt sein.

Das Ventil 7 kann die unterschiedlichsten Formen aufweisen. In den oben beschriebenen Ausführungsformen, insbesondere in Figur 1k, ist ein Ventil 7 offenbart, welches einen zylinderförmigen Ventilkörper 71 und eine diesen Ventilkörper 71 überdeckende Membran 70 aufweist. Der Ventilkörper 71 ist vorzugsweise aus einem starren Material, insbesondere Kunststoff gefertigt. Die Membran 70 besteht aus einem flexiblen Material, vorzugsweise aus Gummi, Kautschuk oder Silikon oder TPE.

Das Ventil 7ist in seiner Lage fixiert, indem der Ventilkörper 71 über einen Stutzen gestülpt ist. Der Ventilkörper 71 weist an einer Stirnseite Durchlassöffnungen 72 und eine mittig angeordnete Aufnahmeöffnung 73 auf. Passend dazu weist die Membran einen vorzugsweise mittig angeordneten Verbindungsknopf 74 auf, welcher zur Befestigung der Membran in die Aufnahmeöffnung 73 einsteckbar ist.

Es lassen sich aber auch andere Ventile in den oben beschriebenen Ausführungsformen des Brusthaubensets einsetzen. So kann, wie dies in Figur 6 dargestellt ist, eine Membranaufnahme 78 in Form eines Verbindungsknopfes am Ventilkörper 71 und die Aufnahmeöffnung 73 in der Membran 70 angeordnet sein.

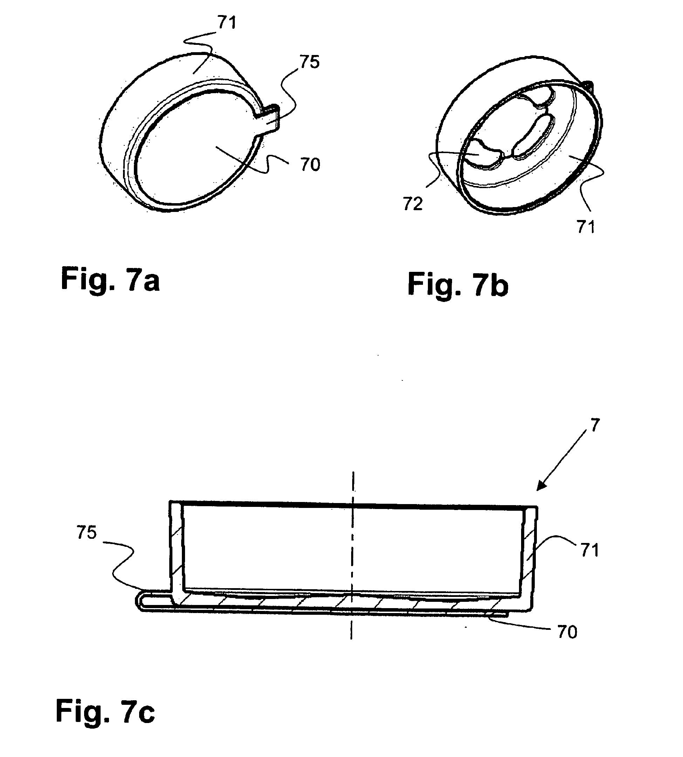

Die Membran 70 kann auch, wie dies in den Figuren 7a bis 7c dargestellt ist, einstückig über ein Scharnier bzw. eine Lippe 75 mit dem Ventilkörper 71 verbunden sein. In diesem Fall besteht das Ventil 7 vorzugsweise aus einem einheitlichen Material, wobei die Membran dünnwandiger als der Ventilkörper ausgebildet ist.

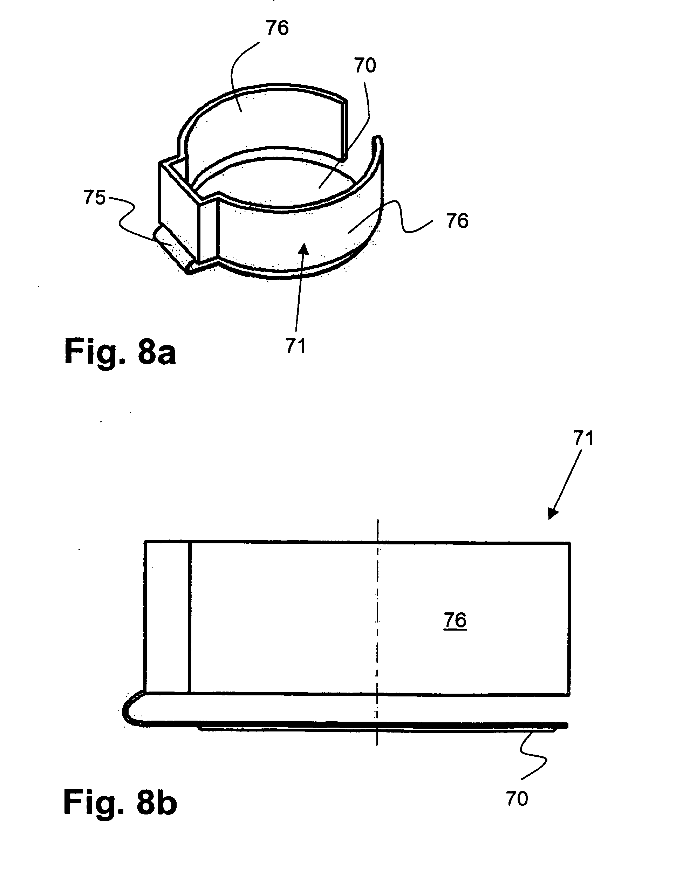

Des weiteren kann der Ventilkörper 71 gemäss den Figuren 8a und 8b nicht mit einem geschlossenen Mantel sondern mit einem einseitig offenen Mantel ausgebildet sein. Dadurch lassen sich die zwei Mantelteile 76 federnd ausbilden, was einerseits die Montage des Ventils 7 erleichtert und andererseits ihren Sitz auf dem Stutzen 30 verbessert.

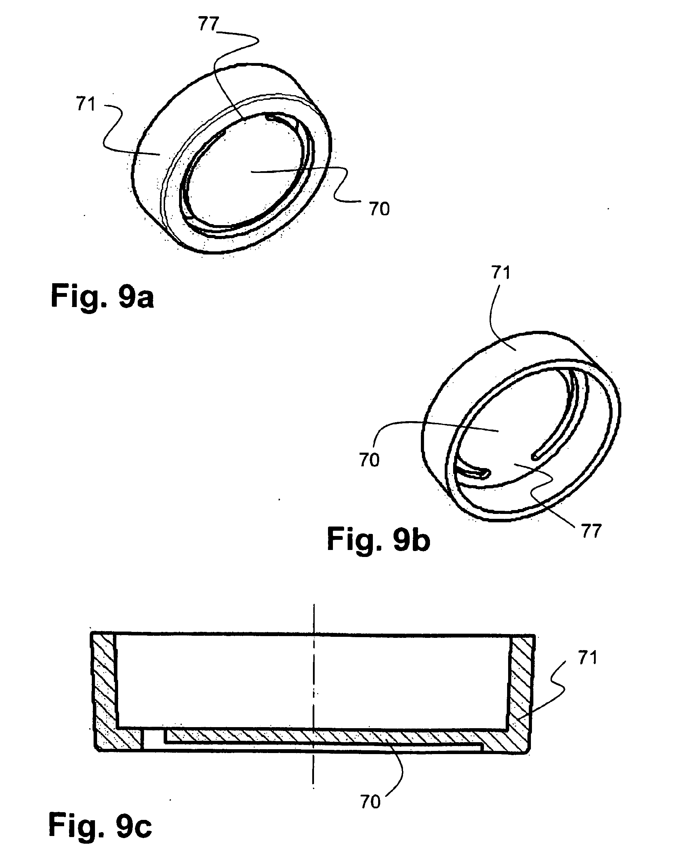

In einer weiteren Ausführungsform gemäss den Figuren 9a bis 9c ist die Membran 70 über einen Steg 77 am Ventilkörper 71 angeformt, wobei die beiden Innenflächen der Membran sowie die inneren Ventilkörperstimflächen in einer Ebene fluchten.

Das erfindungsgemässe Brusthaubenset lässt sich kostengünstig herstellen und dank seiner Einzelteile platzsparend steril verpacken. Es ermöglicht zudem die Verwendung des Sammelbehälters als Babyflasche und verunmöglicht trotzdem eine erneute Verwendung des Sets zum Abpumpen von Muttermilch.

### Bezugszeichenliste

- 1: Sammelbehälter
- 11: Erstes unteres Schnappverschlusselement

- 2: Kopplungsteil
- 20: Erstes oberes Schnappverschlusselement
- 21: Aussengewinde

- 3: Brusthaubenanschlussteil
- 3': Oberes Element
- 3": Unteres Element
- 30: Behälterseitiger Anschlussstutzen
- 31: Trichterseitiger Anschlussstutzen
- 32: Pumpenseitiger Anschlussstutzen
- 33: Zweites oberes Schnappverschlusselement
- 34: Zweites unteres Schnappverschlusselement
- 35: Behälterseitige Einlassöffnung

- 4: Garantieelement
- 40: Lasche
- 41: Steg

- 5: Brusthaubentrichter
- 50: Trichterelement
- 51: Trichterstutzen

- 6: Verbindungsschlauch
- 60: Zweites Ende

- 7: Ventil
- 70: Ventilmembran

- 71: Ventilkörper
- 72: Durchlassöffnungen
- 73: Aufnahmeöffnung
- 74: Verbindungsknopf
- 75: Lippe
- 76: Mantelteil
- 77: Steg
- 78: Membranaufnahme

- 8: Verschlussdeckel

## Patentansprüche

1. Einweg-Brusthaubenset, wobei das Set ein Kopplungsteil (2) zur Verbindung mit einem Milchsammelbehälter (1) und ein Brusthaubenanschlussteil (3) zur Verbindung mit einem Brusthaubentrichter (5) aufweist, wobei Kopplungsteil (2) und Brusthaubenanschlussteil (3) miteinander verbunden sind und wobei sie zwecks Lagerung oder Verabreichung der im Milchsammelbehälter gesammelten Milch zu trennen sind, **dadurch gekennzeichnet, dass** das Kopplungsteil (2) und das Brusthaubenanschlussteil (3) über ein Garantieelement (4) miteinander verbunden sind, welches bei einer erstmaligen Trennung von Kopplungsteil (2) und Brusthaubenanschlussteil (3) oder bei Entfernung des Garantieelements (4) deren Verbindung irreversibel zerstört, so dass das Garantieelement gewährleistet, dass das Kopplungsteil (2) und das Brusthaubenanschlussteil (3) nur ein einziges Mal zum Abpumpen der Milch verwendbar sind.

2. Brusthaubenset nach Anspruch 1, wobei Kopplungsteil (2) und Brusthaubenanschlussteil (3) nur voneinander getrennt werden können, wenn das Garantieelement (4) irreversibel zerstört ist.

3. Brusthaubenset nach Anspruch 1 oder 2, wobei das Kopplungsteil (2) Teil einer Flasche ist, wobei das Kopplungsteil einen Flaschenhals aufweist und wobei das Garantieelement (4) eine Reisssicherung ist, welche am Flaschenhals angeordnet ist.

4. Brusthaubenset nach einem der Ansprüche 1, 2 oder 3, wobei das Garantieelement (4) über zerstörbare Stege (41) mit dem Kopplungsteil (2) und/oder mit dem Brusthaubenanschlussteil (3) verbunden ist.

5. Brusthaubenset nach einem der Ansprüche 1 bis 4, wobei das Garantieelement (4) ein teil- oder vollständig umlaufende Band ist.

6. Brusthaubenset nach einem der Ansprüche 1 bis 5, wobei das Set einen Milchsammelbehälter (1) aufweist, welcher zusammen mit dem Kopplungsteil (2) einstückig ausgebildet ist.

7. Brusthaubenset nach einem der Ansprüche 1 bis 5, wobei das Set einen Milchsammelbehälter (1) aufweist, welcher becherförmig ausgebildet ist, welcher mit dem Kopplungsteil (2) verbindbar ist und welcher zusammen mit dem Kopplungsteil (2) eine verschliessbare Flasche bildet.

8. Brusthaubenset nach Anspruch 7, wobei Kopplungsteil (2) und Sammelbehälter (1) über eine Schnappverschlussverbindung miteinander verbindbar sind.

9. Brusthaubenset nach einem der Ansprüche 1 bis 8, wobei das Kopplungsteil (2) mit einem Aussengewinde (21) versehen ist.

10. Brusthaubenset nach Anspruch 9, wobei das Garantieelement (4) oberhalb des Aussengewindes (21) angeordnet ist.

11. Brusthaubenset nach einem der Ansprüche 1 bis 10, wobei das Brusthaubenanschlussteil (3) ein oberes Element (3') und ein unteres Element (3") aufweist, wobei das obere Element (3') in das untere Element (3") einsteckbar ist und mittels eines Schnappverschlusses (33, 34) fixierbar ist.

12. Brusthaubenset nach Anspruch 11, wobei das untere Element (3 ") mittels des Garantieelements (4) mit dem Kopplungsteil (2) verbunden ist.

13. Brusthaubenset nach Anspruch 10, wobei es ein Einwegventil (7) aufweist, welches eine behälterseitige Einlassöfnung (35) des Brusthaubenanschlussteils (3) verschliesst, wobei das Einwegventil (7) einen mit Durchlassöffnungen (72) versehenen Ventilkörper (71) und eine diese Durchlassöffnung (72) verschliessende Ventilmembran (70) aufweist und wobei der Ventilkörper (71) an einem nach innen ragenden Gegenstück (34) des unteren Elements (3") angeordnet ist.

14. Brusthaubenset nach einem der Ansprüche 1 bis 12, wobei es ein Einwegventil (7) aufweist, welches eine behälterseitige Einlassöffnung (35) des Brusthaubenanschlussteils (3) verschliesst, wobei das Einwegventil (7) einen mit Durchlassöffnungen (72) versehenen Ventilkörper (71) und eine diese Durchlassöffnung (72) verschliessende Ventilmembran (70) aufweist und wobei die Ventilmembran (70) einstückig am Ventilkörper (71) angeformt ist.

## Claims

1. A disposable breast shield set, the set comprising a coupling part (2) for connection to a milk collection receptacle (1), and a breast shield attachment part (3) for connection to a breast shield funnel (5), the coupling part (2) and the breast shield attachment part (3) being connected to one another and wherein they have to be separated from each other in order to store the milk or administer the milk collected in the milk collection receptacle, **characterized in that** the coupling part (2) and the breast shield attachment part (3) are connected to each other via a tamper-evident element (4), this tamper-evident element (4) destroying their connection irreversibly the first time the coupling part (2) and the breast shield attachment part (3) are separated or when the tamper-evident element (4) is removed, so that the tamper-evident element (4) guarantees that the coupling part (2) and the breast shield attachment part (3) can be used just one time for pumping off milk.

2. The breast shield set as claimed in claim 1, wherein the coupling part (2) and breast shield attachment part (3) can be separated from one another only when the tamper-evident element (4) has been irreversibly destroyed.

3. The breast shield set as claimed in claim 1 or 2, wherein the coupling part (2) is part of a bottle, wherein the coupling part has a bottle neck, and wherein the tamper-evident element (4) is a tear-open seal arranged on the bottle neck.

4. The breast shield set as claimed in one of claims 1, 2 or 3, wherein the tamper-evident element (4) is connected to the coupling part (2) and/or to the breast shield attachment part (3) via destructible webs (41).

5. The breast shield set as claimed in one of claims 1 to 4, wherein the tamper-evident element (4) is a band extending round a partial or complete circumference.

6. The breast shield set as claimed in one of claims 1 to 5, wherein the set comprises a milk collection receptacle (1) that is formed in one piece with the coupling part (2).

7. The breast shield set as claimed in one of claims 1 to 5, wherein the set comprises a milk collection receptacle (1) which is beaker-shaped, can be connected to the coupling part (2), and forms a closable bottle together with the coupling part (2).

8. The breast shield set as claimed in claim 7, wherein coupling part (2) and collection receptacle (1) can be connected to one another via a snap-fit closure.

9. The breast shield set as claimed in one of claims 1 to 8, wherein the coupling part (2) is provided with an external thread (21).

10. The breast shield set as claimed in claim 9, wherein the tamper-evident element (4) is arranged above the external thread (21).

11. The breast shield set as claimed in one of claims 1 to 10, wherein the breast shield attachment part (3) has an upper element (3') and a lower element (3"), the upper element (3') being able to be plugged into the lower element (3") and being able to be fixed by means of a snap-fit closure (33, 34).

12. The breast shield set as claimed in claim 11, wherein the lower element (3") is connected to the coupling part (2) by means of the tamper-evident element (4).

13. The breast shield set as claimed in claim 10, wherein it comprises a disposable valve (7) which closes a receptacle-side inlet opening (35) of the breast shield attachment part (3), the disposable valve (7) having a valve body (71) provided with through-openings (72), and a valve membrane (70) that closes these through-openings (72), and wherein the valve body (71) is arranged on an inwardly protruding mating piece (34) of the lower element (3").

14. The breast shield set as claimed in one of claims 1 to 12, wherein it comprises a disposable valve (7) which closes a receptacle-side inlet opening (35) of the breast shield attachment part (3), the disposable valve (7) having a valve body (71) provided with through-openings (72), and a valve membrane (70) that closes these through-openings (72), and wherein the valve membrane (70) is formed integrally on the valve body (71).

## Revendications

1. Ensemble de téterelle jetable, l'ensemble présentant une partie d'accouplement (2) pour le raccordement à un collecteur de lait (1) et une partie de raccordement de téterelle (3) pour le raccordement à un entonnoir de téterelle (5), la partie d'accouplement (2) et la partie de raccordement de téterelle (3) étant raccordées l'une à l'autre et pouvant être séparées en vue du stockage ou de l'administration du lait recueilli dans le collecteur de lait, **caractérisé en ce que** la partie d'accouplement (2) et la partie de raccordement de téterelle (3) sont raccordées l'une à l'autre par le biais d'un élément de garantie (4) qui, lors de la première séparation de la partie d'accouplement (2) et de la partie de raccordement de téterelle (3), ou en cas d'enlèvement de l'élément de garantie (4), détruit leur raccordement de manière irréversible, de sorte que l'élément de garantie garantisse que la partie d'accouplement (2) et la partie de raccordement de téterelle (3) ne puissent être utilisées qu'une seule fois pour pomper le lait.

2. Ensemble de téterelle selon la revendication 1, dans lequel la partie d'accouplement (2) et la partie de raccordement de téterelle (3) ne peuvent être séparées l'une de l'autre qu'après la destruction irréversible de l'élément de garantie (4).

3. Ensemble de téterelle selon la revendication 1 ou 2, dans lequel la partie d'accouplement (2) fait partie d'une bouteille, la partie d'accouplement présentant un goulot de bouteille et l'élément de garantie (4) étant une fixation arrachable, qui est disposée sur le goulot de la bouteille.

4. Ensemble de téterelle selon la revendication 1, 2 ou 3, dans lequel l'élément de garantie (4) est connecté par le biais de nervures destructibles (41) à la partie d'accouplement (2) et/ou à la partie de raccordement de téterelle (3).

5. Ensemble de téterelle selon l'une quelconque des revendications 1 à 4, dans lequel l'élément de garantie (4) est une bande entourant toute ou une partie de la circonférence.

6. Ensemble de téterelle selon l'une quelconque des revendications 1 à 5, dans lequel l'ensemble présente un collecteur de lait (1) qui est réalisé d'une seule pièce avec la partie d'accouplement (2).

7. Ensemble de téterelle selon l'une quelconque des revendications 1 à 5, dans lequel l'ensemble présente un collecteur de lait (1) qui est réalisé en forme de gobelet, qui peut être raccordé à la partie d'accouplement (2) et qui forme, conjointement avec la partie d'accouplement (2), une bouteille pouvant être fermée.

8. Ensemble de téterelle selon la revendication 7, dans lequel la partie d'accouplement (2) et le collecteur (1) peuvent être raccordés l'un à l'autre par le biais d'une connexion à fermeture par encliquetage.

9. Ensemble de téterelle selon l'une quelconque des revendications 1 à 8, dans lequel la partie d'accouplement (2) est pourvue d'un filetage extérieur (21).

10. Ensemble de téterelle selon la revendication 9, dans lequel l'élément de garantie (4) est disposé au-dessus du filetage extérieur (21).

11. Ensemble de téterelle selon l'une quelconque des revendications 1 à 10, dans lequel la partie de raccordement de téterelle (3) présente un élément supérieur (3') et un élément inférieur (3"), l'élément supérieur (3') pouvant être enfiché dans l'élément inférieur (3") et pouvant être fixé au moyen d'une fermeture par encliquetage (33, 34).

12. Ensemble de téterelle selon la revendication 11, dans lequel l'élément inférieur (3") est raccordé à la partie d'accouplement (2) au moyen de l'élément de garantie (4).

13. Ensemble de téterelle selon la revendication 10, présentant une valve unidirectionnelle (7), qui ferme une ouverture d'entrée (35) côté récipient de la partie de raccordement de téterelle (3), la valve unidirectionnelle (7) présentant un corps de valve (71) pourvu d'ouvertures de passage (72) et une membrane de valve (70) fermant cette ouverture de passage (72), le corps de valve (71) étant disposé sur une pièce opposée saillant vers l'intérieur (34) de l'élément inférieur (3").

14. Ensemble de téterelle selon l'une quelconque des revendications 1 à 12, présentant une valve unidirectionnelle (7), qui ferme une ouverture d'entrée (35) côté récipient de la partie de raccordement de téterelle (3), la valve unidirectionnelle (7) présentant un corps de valve (71) pourvu d'ouvertures de passage (72) et une membrane de valve (70) fermant cette ouverture de passage (72) et la membrane de valve (70) étant façonnée d'une seule pièce sur le corps de valve (71).
